(19) Europäisches Patentamt
European Patent Office
Office européen des brevets

(11) Veröffentlichungsnummer: **0 211 805**
**A2**

(12) **EUROPÄISCHE PATENTANMELDUNG**

(21) Anmeldenummer: 86810326.8

(22) Anmeldetag: 18.07.86

(51) Int. Cl.⁴: C 07 D 487/04
C 07 D 209/48, A 01 N 43/38
A 01 N 43/90

(30) Priorität: 24.07.85 CH 3222/85

(43) Veröffentlichungstag der Anmeldung:
25.02.87 Patentblatt 87/9

(84) Benannte Vertragsstaaten:
BE CH DE FR GB IT LI NL

(71) Anmelder: CIBA-GEIGY AG
Klybeckstrasse 141
CH-4002 Basel(CH)

(72) Erfinder: Böhner, Beat, Dr.
Hügelweg 3
CH-4102 Binningen(CH)

(72) Erfinder: Moser, Hans, Dr.
Maispracherstrasse 12
CH-4312 Magden(CH)

(72) Erfinder: Pissiotas, Georg, Dr.
Am Sonnenrain 71
CH-7850 Lörrach(CH)

(54) **Neue N-(2-Fluorphenyl)-azolidine.**

(57) Neue N-(2-Fluorphenyl)-azolidine der untenstehenden Formel I haben gute pre- und postemergente selektivherbizide Eigenschaften ferner beeinflussen respektive hemmen sie das Pflanzenwachstum. Die Verbindungen entsprechen der Formel I

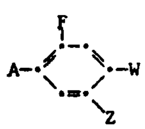

(I)

worin

A einen der bicyclischen Reste

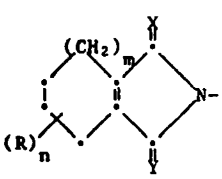

,

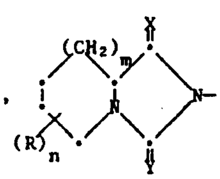

oder

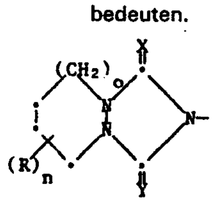

darstellt,

m Null oder eins

n Null, eins oder zwei

o Null, eins, zwei oder drei,

R $C_1$-$C_4$ Alkyl oder Halogen

W Halogen

X und Y unabhängig voneinander Sauerstoff oder Schwefel

Z $C_1$-$C_4$ Halogenalkoxy, $C_1$-$C_4$ Halogenalkylthio, $C_2$-$C_6$ Halogenalkenyloxy, $C_2$-$C_6$ Halogenalkenylthio, $C_3$-$C_6$ Halogenalkinyloxy oder $C_3$-$C_6$ Halogenalkinylthio bedeuten.

EP 0 211 805 A2

Croydon Printing Company Ltd.

0211805

CIBA-GEIGY AG
Basel (Schweiz)

5-15440/-

# Neue N-(2-Fluorphenyl)-azolidine

Die vorliegende Erfindung betrifft neue N-(2-Fluorphenyl)-azolidine der untenstehenden Formel I mit herbizider und den Pflanzenwuchs regulierender Wirkung, sowie die Herstellung dieser neuen Verbindungen. Ferner betrifft sie die Mittel, welche die neuen Verbindungen enthalten sowie deren Verwendung zur selektiven Bekämpfung von Unkräutern oder zur Regulierung des Pflanzenwuchses.

Die neuen N-(2-Fluorphenyl)-azolidine entsprechen der Formel I

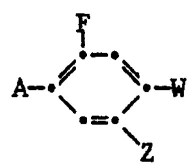 (I)

worin A einen der bicyclischen Reste

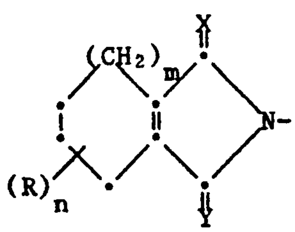 , 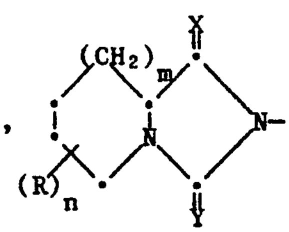 oder 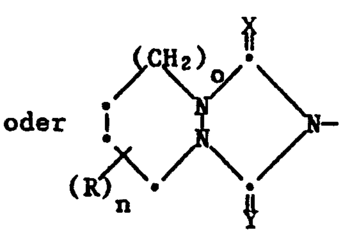

darstellt
m Null oder eins
n Null, eins oder zwei
o Null, eins zwei oder drei
R $C_1$-$C_4$-Alkyl oder Halogen
W Halogen
X und Y unabhängig voneinander Sauerstoff oder Schwefel
Z $C_1$-$C_4$ Halogenalkoxy, $C_1$-$C_4$ Halogenalkylthio, $C_2$-$C_6$ Halogenalkenyloxy, $C_2$-$C_6$ Halogenalkenylthio, $C_3$-$C_6$ Halogenalkinyloxy oder $C_3$-$C_6$ Halogenalkinylthio
bedeuten.

In der Formel I ist unter Halogen, Fluor, Chlor, Brom oder Iod zu verstehen. Beim Substituenten W ist Fluor, Chlor und Brom bevorzugt.

Der Ausdruck Alkyl allein oder als Teil eines Substituenten umfasst verzweigte oder geradkettige Alkylgruppen. Beispiele sind Methyl, Aethyl, n-Propyl, Isopropyl, n-Butyl, Isobutyl, sek.-Butyl, tert.-Butyl, n-Pentyl, Isopentyl, Neopentyl sowie Hexyl mit seinen Isomeren.

Die Alkenyl- und Alkinylreste können ebenfalls verzweigt oder geradkettig sein und umfassen vorzugsweise 2-6 Kohlenstoffatome. Beispiele sind Allyl, Methallyl, Butenyl, Butadienyl, Methylbutenyl, Dimethylbutenyl, Pentenyl, Hexenyl, Pentendienyl und Hexendienyl, Butin, Methylbutin, Pentin, Hexin, Dimethylbutin.

Die dem Rest A entsprechenden bicyclischen Ringsysteme umfassen die folgenden Grundkörper:

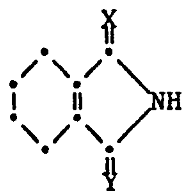

3,4,5,6-Phthalsäureimid und die entsprechenden Thiophthalsäure-imide

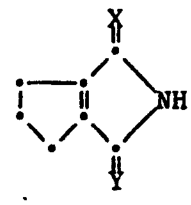

Cyclopent-1-en-1,2-dicarbon-säureimid und die entsprechenden Thiosäureimide

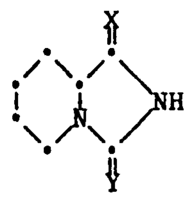

7,9-Dioxo-1,8-diazabicyclo-[4.3.0]nonan und seine 7 und/oder 9-Thion-analogen

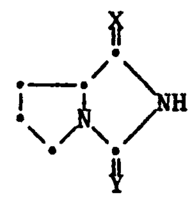

6,8-Dioxo-1,7-diazabicyclo-[3.3.0]octan und seine 6 und/oder 8-Thion-analogen.

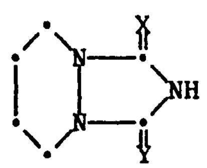

7,9-Dioxo-1,6,8-triazabicyclo-[4.3.0]nonan und seine 7 und/oder 9-on-analogen

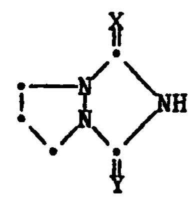

6,8-Dioxo-1,5,7-triazabicyclo-[3.3.0]octan und seine 6 und/oder 8-Thion-analogen

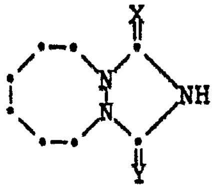

9,11-Dioxo-1,8,10-triazabicyclo-[6.3.0]undecan und seine 9- und/oder 11-Thion-analogen

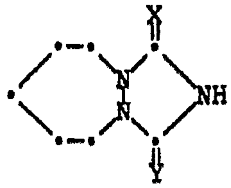

8,10-Dioxo-1,7,9-triazabicyclo-[5.3.0]decan und seine 8 und/oder 10-Thion-analogen.

Die erfindungsgemässen N-(2-Fluorphenyl)-azolidine können je nach der Art der zugrundeliegenden Bicycloheterocyclus folgenden Formeln zugeordnet werden.

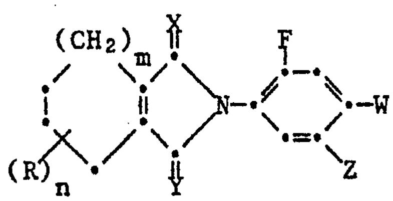

Ia

In dieser Formel bedeutet m Null oder eins während n R, W, X, Y und Z die unter der Formel I gegebene Bedeutung haben.

Gute Wirkung zeigen diejenigen Verbindungen, in denen m eins, n Null, X und Y je Sauerstoff, W Chlor sind und Z eine der unter der Formel I gegebene Bedeutungen hat, besonders jedoch die Verbindungen:

N-(2-Fluor-4-chlor-5-difluormethoxyphenyl)-3,4,5,6-tetrahydrophthalsäureimid,

N-[2-Fluor-4-chlor-5-(2-chlorprop-2-en-1-yloxy)-phenyl]-3,4,5,6-tetrahydrophthalsäureimid,

N-[2-Fluor-4-chlor-5-(2-chlor-1,1,2-trifluoräthoxy)-phenyl]-3,4,5,6-tetrahydrophthalsäureimid.

Erfindungsgemässe Verbindungen entsprechen ferner der Formel

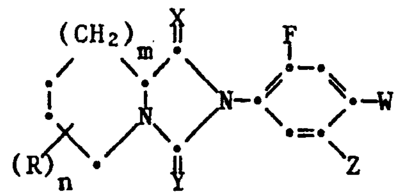

Ib

In dieser Formel bedeutet m Null oder eins während n, R, W, X, Y und Z die unter der Formel I gegebene Bedeutung haben.

Gute Wirkung zeigen diejenigen Verbindungen, in denen m eins, n Null, W Chlor, X und Y je Sauerstoff sind und Z eine der unter der Formel I gegebenen Bedeutungen hat.

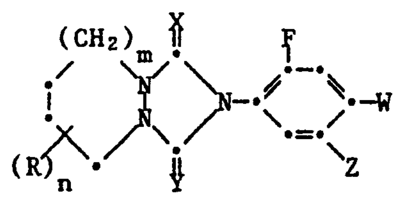

Ic

In dieser Formel bedeutet m Null oder eins während n, R, W, X, Y und Z die unter der Formel I gegebene Bedeutungen haben. Gute Wirkung zeigen diejenigen Verbindungen, in denen m eins, n Null, W Chlor, X und Y je Sauerstoff sind und Z eine der unter der Formel I gegebenen Bedeutungen hat.

Die N-(2-Fluorphenyl)-azolidin-Verbindungen der Formel I können auf verschiedene an sich bekannte Weise hergestellt werden.

Ein erstes, allgemeingültiges Verfahren, welches sich vorzugsweise für die Umsetzung mit einem Tetrahydrophthalsäureimid eignet, besteht darin, dass man das Anhydrid einer bicyclischen Dicarbonsäure entsprechend der Definiton des Restes A der Formel II

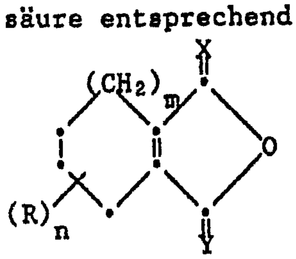

,

(II)

worin R, n, X und Y die unter der Formel I gegebene Bedeutung haben, gegebenenfalls in einem inerten organischen Lösung- oder Verdünnungsmittel mit der äquimolaren Menge eines 2-Fluoranilins der Formel III kondensiert

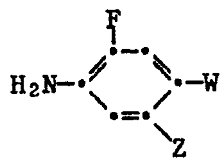

(III),

worin W und Z die in Anspruch 1 gegebene Bedeutung haben, und das entstandene Amid der Formel IV

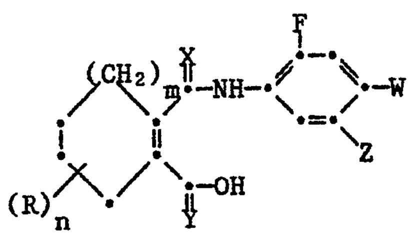

,

(IV)

worin m, n, o, R, W, X, Y und Z die unter der Formel I gegebene Bedeutung haben, gegebenenfalls ohne es aus dem Reaktionsgemisch zu isolieren, bei einer Temperatur zwischen 80 und 230°C ringschliesst.

Als Lösungsmittel kommen unter diesen Bedingungen Kohlenwasserstoffe, Aether, Alkohole, Nitrile und Säuren wie Essig- oder Propionsäure in Frage, deren Siedepunkt mindestens 80° beträgt, wie z.B. Hexan, Cyclohexan, Toluol, Xylol, Tetrahydrofuran, Diisopropyläther, Butanol, Propanol, Acetonitril.

Die Temperatur, welche zum Ringschluss führt, liegt zwischen 80 und 230°C, vorzugsweise zwischen 100-180°C. Praktisch bedeutet dies Siedetemperatur des Reaktionsgemisches.

Ein erfindungsgemässes Verfahren zur Herstellung der N-(2-Fluorphenyl)-azolidine der Formel Ia besteht darin, dass man ein Säureanhydrid der Formel II

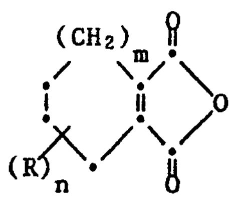

(II)

worin m Null oder eins bedeutet und n, R und W die unter der Formel I gegebene Bedeutung haben, gegebennfalls in einem inerten organischen Lösungsmittel mit der äquimolaren Menge eines 2-Fluoranilins der Formel III kondensiert

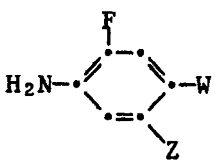

(III)

worin W und Z die unter der Formel I gegebene Bedeutung haben, und das entstandene Anilid der Formel IVa

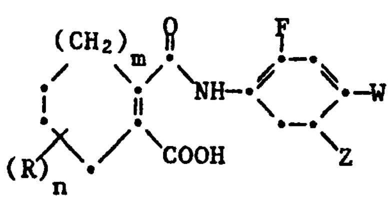

(IVa)

worin m Null oder eins bedeutet und n, R, W und Z die unter der Formel I gegebene Bedeutung haben, gegebenenfalls ohne es aus dem Reaktionsgemisch zu isolieren, bei einer Temperatur von 80-230°C ringschliesst.

Die Verbindungen der Formeln IV und IVa sind neue Zwischenprodukte und ebenfalls Gegenstand dieser Erfindung.

Die N-(2-Fluorphenyl)-azolidin-Verbindungen der Formel Ib werden zweckmässigerweise durch Kondensation einer 2-Piperidin-carbonsäure oder einem oder zwei Ringkohlenstoffatomen durch $C_1$-$C_4$ Alkyl oder Halogen substituiert sein können mit dem Isocyanat oder Isothiocyanat eines 2-Fluoroanilides und Ringschluss des entstandenen N-(2-Carboxyl-piperidino- oder pyrrolidino)-N-(2-fluorphenyl)-harnstoffes oder -thioharnstoffes bei höherer Temperatur.

Ein Verfahren zur Herstellung der N-(2-Fluorphenyl)-azolidine der Formel Ib ist dadurch gekennzeichnet, dass man in einem inerten organischen Lösungsmittel in Gegenwart einer Base das Derivat eines 2-Carboxylpiperidins oder 2-Carboxylpyrrolidins der Formel V

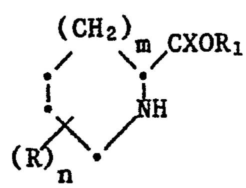

(V)

worin m Null oder eins und $R_1$ Wasserstoff, $C_1$-$C_6$ Alkyl oder Benzyl bedeutet, und n, R und X die unter der Formel I gegebene Bedeutung haben, bei einer Temperatur von 0-150°C mit dem Isocyanat oder Isothiocyanat eines 2-Fluoranilides der Formel VI kondensiert

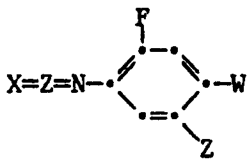

VI

worin W, X und Z die unter der Formel I gegebene Bedeutung haben, und das entstandene Harnstoffderivat der Formel VII

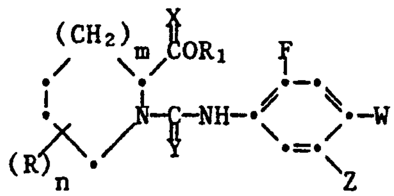

(VII)

worin m Null oder 1 und $R_1$ Wasserstoff $C_1$-$C_6$ Alkyl oder Benzyl bedeuten, während n, R, W, X, Y und Z die unter der Formel I gegebene Bedeutung haben, gegebenenfalls ohne es aus dem Reaktionsgemisch zu isolieren in saurem Milieu bei einer Temperatur zwischen 50 und 150°C ringschliesst.

Die Kondensation findet in einem inerten organischen Lösungsmittel bei einer Temperatur von 0-150°C vorzugsweise 20 bis 100°C statt. Als Lösungsmittel kommen unter den Reaktionsbedingungen inerte flüssige Kohlenwasserstoffe, Aether, Alkohole, Ketone, Nitrile in Frage. Als Beispiele seien Benzol, Toluol, Xylol, Hexan, Cyclohexan, Diäthyläther, Diisopropyläther, Tetrahydrofuran, Dioxan, Aethanol, Isopropanol, Butanol, Aceton, Methyläthylketon und Acetonitril genannt.

Die Reaktion verläuft auch in Wasser oder wasserhaltigen Lösungsmitteln, wenn ein Isocyanat der Formel VI verwendet wird. Die Kondensation wird in Gegenwart einer Base, beispielsweise Natronlauge vorgenommen.

Die Ringschlussreaktion verläuft bei höherer Temperatur, zwischen 50 und 150°C am besten bei der Rückflusstemperatur des Reaktionsgemisches in saurem Milieu. Dabei können dieselben Lösungsmittel wie bei der Kondensation verwendet werden, oder auch z.B. auch Essig- oder Propionsäure. Das Reaktionsmilieu muss zum Ringschluss sauer gestellt werden, was wenn das Harnstoffderivat der Formel VII nicht isoliert wird am besten durch Zugabe einer starken Säure wie Salz- oder Schwefelsäure in das Reaktionsgemisch geschieht.

Die Harnstoffderivate der Formel VII sind neue Zwischenprodukte und ebenfalls Gegenstand dieser Erfindung.

Um die 1-(2-Fluorphenyl)-1H-1,3,4-triazolidinverbindungen der Formel Ic herzustellen, bieten sich verschiedene Synthesewege an.

Gemäss einem ersten Verfahren wird ein 1-(2-Fluorphenyl)-1H-1,3,4-triazolidin-2,5-dionderivat der Formel VIII

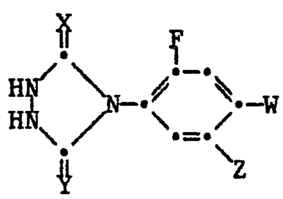

(VIII)

worin W, X, Y und Z die unter der Formel I gegebene Bedeutung haben, in Gegenwart einer Base oder eines Metallsalzes der obigen Verbindung, in einem inerten organischen Lösungsmittel, bei einer Temperatur, die zwischen -20° und +200°C liegt, mit einer Verbindung der Formel

U-B-U (IX)
$(R)_n$

worin B eine $C_3$-$C_6$ Alkylenkette und U eine reaktive Abgangsgruppe bedeutet während n und R die im Anspruch 1 gegebene Bedeutung haben, kondensiert.

Als Lösungsmittel kommen unter den Reaktionsbedingungen inerte Flüssigkeiten wie Wasser, Alkohole wie Methanol, Aethanol, Propanol, Isopropanol oder Butanol, Kohlenwasserstoffe wie Benzol, Toluol, Xylol, Hexan oder Cyclohexan, Aether wie Diisopropyläther, Tetrahydrofuran oder Dioxan, Ketone wie Aceton, Methyläthylketon, ferner Dimethylformamid, Dimethylsulfoxid, Acetonitril, Hexametapol, sowie Dimethylacetamid in Frage.

Als Basen können Natriumhydroxyd, Kaliumhydroxid, Natriumhydrid, Natriumcarbonat, Kaliumcarbonat, ein Natriumalkoholat oder Kaliumalkoholat sowie organische Verbindungen, wie Pyridin, Collidin, Lutidin oder Trialkylamine, z.B. Triäthylamin eingesetzt werden. Man kann die Verbindung der Formel VIII, welche ein Urazol ist auch als Dinatrium- oder Dikaliumsalz in die Reaktion geben.

Die Verbindung der Formel IX ist eine gegebenenfalls durch $(R)_n$ substituiertes $C_3$-$C_8$-Alkylen und ist mit zwei Abgangsgruppen U versehen. Als solche kommen Esterreste wie Halogenatome, Mesyl- oder Tosylreste sowie Alkylcarbonyloxyreste in Frage. Geeignete Verbindungen der Formel IX sind z.B. 1,3-Dihalogenpropan, 1,4-Dihalogenbutan, 1,4-Dihalogen-2-methylbutan, 1,4-Dihalogenpentan, 2,5-Dihalogenhexan, 2,4-Dihalogenhexan, 1,3-Dihalogenpentan, 1,3-Ditosylpropylen, 1,4-Dimesitylbutylen.

Die Reaktion findet bei Temperaturen die zwischen -20° und +200°C liegen, vorzugsweise zwischen 0 und 140°C statt. Im allgemeinen wird die Reaktion unter Rühren bei Raumtemperatur angefangen und am Schluss für kurze Zeit bis zum Siedepunkt des Reaktionsgemisches erhitzt.

Ein Weg zur Herstellung der 1-(2-Fluorphenyl)-1H-1,3,4-triazolidin-2-on-4-thione der Formel Ic besteht darin, dass man das Isocyanat oder Isothiocyanat eines 2-Fluoranilins der Formel VI

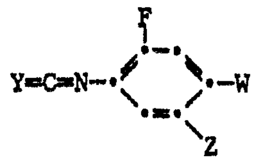 (VI)

worin W, Y und Z die unter der Formel I gegebene Bedeutung haben in einem inerten organischen Lösungsmittel, bei einer Temperatur, die zwischen 0° und 150°C liegt, mit einem Tetrahydropyrazol oder Hexahydropyridazin der Formel X kondensiert,

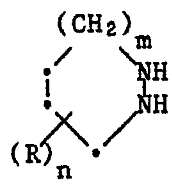 (X)

worin m Null oder 1 bedeutet und n und R die unter der Formel I gegebene Bedeutung haben, und das entstandene Harnstoffderivat der Formel XI

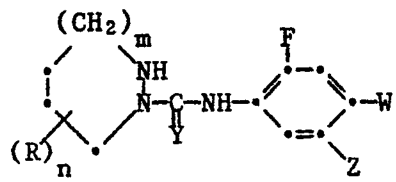 (XI)

worin m Null oder eins bedeutet und n, R, W, Y und Z die unter der Formel I gegebene Bedeutung haben, gegebenenfalls ohne es aus dem Reaktionsgemisch zu isolierien, in einem inerten Lösungsmittel bei einer Temperatur die zwischen 0° und 150°C liegt, in Gegenwart einer anorganischen Base mit Schwefelkohlenstoff zu einer Verbindung der Formel Ic, worin X Schwefel bedeutet umsetzt.

Als Lösungsmittel kommen für diese Umsetzungen inerte polare mit Wasser mischbare Flüssigkeiten wie Alkohole oder Dimethylformamid in Frage. Die Temperaturen liegen zwischen 0° und 150°C vorzugsweise bei 20° und 100°. Auch hier beginnt man die Reaktion im Allgemeinen bei Raumtemperatur und erwärmt am Ende bis zum Siedepunkt des

Reaktionsgemisches. Während der Zugabe von Schwefelkohlenstoff stellt man das Reaktionsgemisch mit einer anorganischen Base, z.B. Natriumhydroxid, Kaliumhydroxid oder Ammoniumhydroxid alkalisch.

Die Zwischenprodukte der Formel XI sind neu und bilden ebenfalls einen Teil dieser Erfindung.

Die 1-(2-Fluorphenyl)-1H-1,3,4-triazolidin-2,5-dion oder -2-on-5-thion-Verbindungen der Formel Ic in denen X Sauerstoff bedeutet, können beispielsweise hergestellt, indem man das Isocyanat oder Isothiocyanat eines 2-Fluoranilides der Formel VI wie oben angegeben mit einem Tetrahydropyrazol oder Hexahydropyridazin der Formel X kondensiert und das entstandene Harnstoffderivat der Formel XI in Gegenwart einer Base mit einem Chlorkohlensäureester der Formel XII

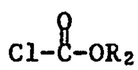

XII

worin $R_2$ $C_1$-$C_6$-Alkyl oder Benzyl bedeutet, kondensiert und dass entstandene Harnstoffderivat der Formel XIII

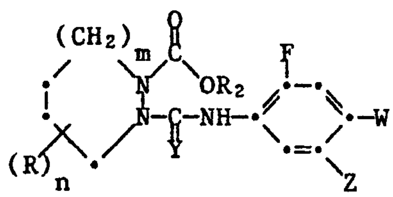

(XIII)

in einem inerten Lösungsmittel, in Gegenwart einer Base bei einer Temperatur von 50°-180° ringschliesst.

Man kann die 1-(2-Fluorphenyl)-1H-1,3,4-triazolidin-2,5-dion oder -2-on-5-thion-Verbindungen der Formel Ic, worin X Sauerstoff ist, auch herstellen, indem man ein Tetrahydropyrazol oder Hexahydropyridazol der Formel X in einem organischen Lösungsmittel in Gegenwart einer Base zuerst mit der äquimolaren Menge eines Chlorkohlensäureesters der Formel XII kondensiert und das entstandene Amid der Formel XIV

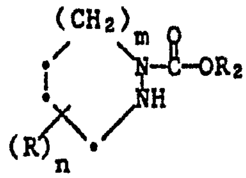

(XIV)

worin m Null oder 1, $R_2$ einen $C_1$-$C_6$ Alkyl oder den Benzylrest bedeuten und n und R die unter der Formel I gegebene Bedeutung hat, weiter mit dem Isocyanat oder Isothiocyanat eines 2-Fluoranilides der Formel VI umsetzt und das entstandene Harnstoffderivat der Formel XIII gegebenenfalls ohne es aus dem Reaktionsgemisch zu isolieren, in einem inerten Lösungsmittel in Gegenwart einer Base bei einer Temperatur die zwischen 0° und 150°C liegt zum Ringschluss bringt.

Diese Synthesewege können wie folgt in einem Schema dargestellt werden:

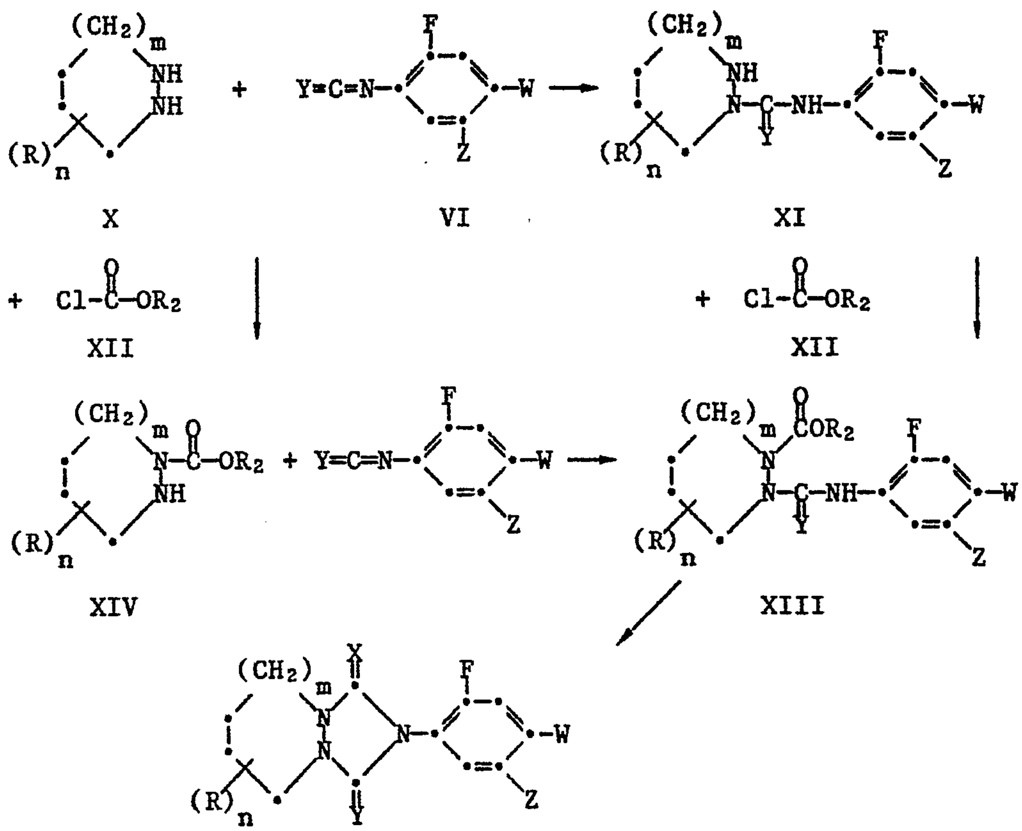

X

VI

XI

XII

XII

XIV

XIII

Ic worin X = Sauerstoff

Das erfindungsgemässe Verfahren zur Herstellung der 1-(2-Fluorphenyl)-1H-1,3,4-triazolidin-2,5-dion- oder -2-on-5-thion-Verbindungen, der Formel Ic, worin X Sauerstoff ist, ist dadurch gekennzeichnet, dass man ein Harnstoffderivat der Formel XIII

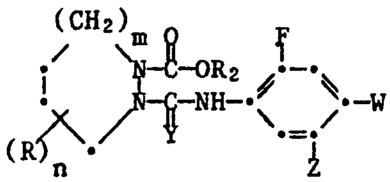

(XIII)

worin m Null oder eins, $R_2$ einen $C_1$-$C_6$ Alkyl- oder Benzylrest bedeuten und n, R, W, Y und Z, die unter der Formel I gegebene Bedeutung haben, und welches in situ hergestellt sein kann, in einem inerten organischen Lösungsmittel, in Gegenwart einer anorganischen Base bei einer Temperatur, die zwischen 0° und 150°C liegt zum Ringschluss bringt.

Als Lösungsmittel kommen für diese Kondensations- und Ringschlussreaktionen den Reagentien gegenüber inerte Flüssigkeiten wie z.B. Wasser, höhersiedende Alkohole wie Aethanol, Propanol, Isopropanol, Butanol, Kohlenwasserstoffe wie Toluol, Xylol, Hexan oder Cyclohexan, Aether wie Diisopropyläther, Dioxan oder Tetrahydrofuran, Ketone wie Methyläthylketon oder Cyclohexanon aber auch andere wie Acetonitril, Dimethylformamid, Dimemthylsulfoxid, Hexametapol, Dimethylacetamid in Frage.

Der Ringschluss findet bei höherer Temperatur, zwischen 50° und 180°C, vorzugsweise bei 70° bis 120°C statt im allgemeinen bei Siedepunkt des Reaktionsgemisches und unter alkalischen Bedingungen statt. Das Reaktionsgemisch wird durch Zugabe einer anorganischen Base, wie Natriumhydroxid, Kaliumhydroxid oder Ammoniak alkalisch gestellt. Das Harnstoffderivat der Formel XIII kann, aber muss nicht isoliert werden, bevor man es dem Ringschluss unterwirft.

Die Harnstoffderivate der Formel XIII sind neue Zwischenprodukte die ebenfalls einen Gegenstand dieser Erfindung bilden.

Eine Verbindung der Formel I in der X und Y Sauerstoff bedeuten kann durch Erhitzen mit einem Schwefel einführenden Mittel wie z.B. Phosphorpentasulfid oder dem Lawesson-Reagens in einem geeigneten inerten organischen Lösungsmittel wie Toluol, Xylol, Dimethylformamid in Gegenwart einer Base oder in einem basischen Lösungsmittel wie Pyridin oder Picolin zur entsprechenden Triono- oder Dithionoverbindung umgesetzt werden in der X und/oder Y Schwefel bedeuten. Je nach der Dosierung des Schwefel einführenden Reagens und den Reaktionsbedingungen können so entweder nur eine oder beide Sauerstoffatome durch Schwefel ersetzt werden.

Das Verfahren zur Herstellung von N-(2-Fluorphenyl)-azoldithiol-Verbindungen der Formel I in denen X und/oder Y Schwefel bedeuten, ist dadurch gekennzeichnet, dass man eine Verbindung der Formel I in der X und/oder Y Sauerstoff bedeutet in einem inerten organischen Lösungsmittel mit einem Schwefel abgehenden Mittel erhitzt.

Die 2-Fluoraniline der Formel II wie auch die N-(2-Fluorphenyl)-azolidin-Verbindungen der Formel I können des weiteren hergestellt werden, indem man ein para 2-Fluor Phenol oder ein para Fluorthiophenol der Formel XVI

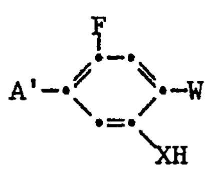

(XVI)

worin A' für die Aminogruppe oder dem unter der Formel I definierten Rest A steht und W und X die unter der Formel I gegebene Bedeutung haben in einem den Reaktionspartnern gegenüber inerten Lösungsmittel in Gegenwart einer Base bei einer Temperatur die zwischen -10° und 140°C liegt mit einer Verbindung der Formel XVII

U - Z XVII,

worin U eine reaktive Abgangsgruppe, wie z.B. ein Halogenatom, einen Mesyl- oder Tosylrest bedeutet und Z die unter der Formel I gegebene Bedeutung hat, reagieren lässt.

Diese Halogenalkylierung bzw. Halogenalkenilierung oder Halogenalkinilierung findet in einem den Reaktionsteilnehhmern gegenüber inerten Lösungsmittel statt. Als solche eignen sich Wasser, Alkanole, Kohlenwasserstoffe, Aether, Ketone, wie z.B. Aethanol, Propanol, Butanol, Benzol, Toluol, Xylol, Cyclohexan, Hexan, Diäthyläther, Diisopropyläther, Tetrahydrofuran, Dioxan, Aceton, Methyläthylketon, Cyclohexanon, aber auch andere wie Acetonitril, Dimethylacetamid, Dimethylformamid und Dimethylsulfoxid.

Die Reaktion findet in Gegenwart der mindestens äquilimolaren Menge einer Base statt. Als solche eignen sich Carbonate wie z.B. Natrium- und Kaliumcarbonat, die Hydroxide von Natrium, Kalium und Ammonium, Natriumhydrid oder auch Kalium- oder Natriumalkoholate, zudem sekundäre oder tertiäre Amine wie z.B. Triäthylamin, Pyridin oder Diäthylamin.

Die Temperatur für diese Reaktion liegt zwischen -10° und +140°C, vorzugsweise zwischen 10° und 100°C. Im Normalfall beginnt man die Temperatur bei Raumtemperatur und erhitzt bei Bedarf gegen Schluss kurz bis zum Siedepunkt des Reaktionsgemisches.

Viele der als Ausgangsmaterial benötigten para Fluorophenole und para Fluorothiophenole sind bekannt oder können analog den beschriebenen Verbindungen hergestellt werden.

Einige dieser Verbindungen zeigen auch bakterizide Wirkung oder Wirkung gegen phytopathogene Pilze und eignen sich zur Behandlung von Saatgut, Früchten, Knollen, etc. zum Schutz vor Infektionen.

Die Verbindungen der Formel I zeigen auch Abzissionswirkung und können zur Erleichterung der Ernte von Obst, Citrusfrüchten, Beeren, Oliven, Nüssen etc. eingesetzt werden. Ihre Dessiccations- und

Defoliationswirkung wird benutzt, um bei Kulturen wie Baumwolle und Kartoffeln kurz vor der Ernte nicht erwünschte vegetative Pflanzenteile auszutrocknen um dadurch die Ernte zu erleichtern.

Hauptsächlich haben die Verbindungen der Formel I jedoch herbizide Wirkung und eignen sich zur Unkrautbekämpfung, besonders von dikotylen Unkräutern. Dabei hat es sich herausgestellt, dass mit diesen Verbindungen die sonst sehr resistenten Problemunkräuter der Familie Galium, Labkrautgewächse, wie z.B. Galium verum, echtes Labkraut, Galium aparine, Kleblabkraut, Galium mollugo, gemeinsames Labkraut etc., gegen welche andere bekannte Herbizide oft nur eine ungenügende Wirkung zeigen, vernichtet werden können.

Ein grosser Vorteil ist dabei, dass die neuen Verbindungen der Formel I sich gegenüber vielen Kulturpflanzen, wie Getreide , Mais oder Reis, aber auch Raps selektiv verhalten und zur Unkrautbekämpfung in solchen Kulturen eingesetzt werden können.

Die Wirkstoffe der Formel I werden in der Regel mit Aufwandmengen von 0,005 bis 4 kg/ha insbesondere 0,001 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen

in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung einiger Verbindungen der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

Beispiel 1

Herstellung von 2-Fluor-4-chlor-5-difluormethoxyanilin (Ausgangsmaterial)

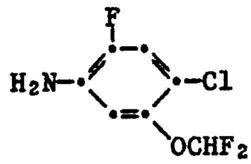

a) Zu einer Lösung von 19,2 g (0,1 Mol) 2-Chlor-4-fluor-5-nitrophenol in 120 ml Dioxan gibt man bei Raumtemperatur unter gutem Rühren 23 ml (0,23 Mol) 10 M Natronlauge. Die Mischung wird auf 50° erwärmt und in die resultierende Suspension langsam Monochlordifluormethan (Freon) eingeleitet. Nach der Sättigung mit Freon wird

das Reaktionsgemisch bei Raumtemperatur noch eine halbe Stunde nachgerührt, dann auf Eiswasser gegossen und mit Aether extrahiert. Die vereinigten Aetherextrakte werden mit Wasser neutral gewaschen und über Natriumsulfat getrocknet. Das nach dem Abdestillieren des Aethers zurückbleibende gelbe Oel (23,5 g) wird am Hochvakuum fraktioniert destilliert. Man erhält 16,3 g 2-Fluor-4-chlor-5-difluormethoxy-nitrobenzol mit einem Siedepunkt von 71°-73°C (0,08 Torr).

b) 33,2 g (0,137 Mol) 2-Fluor-4-chlor-5-difluormethoxy-nitrobenzol werden in 340 ml Tetrahydrofuran gelöst, mit 7 g Raney-Nickel versetzt und bei einer Temperatur von 20-25°C und Normaldruck mit Wasserstoff behandelt. Nach ca. 15 Stunden kommt die Hydrierung zum Stillstand, wobei die theoretische Menge Wasserstoff aufgenommen ist. Man filtrierlt vom Katalysator ab, dampft das Filtrat am Vakuum ein und destilliert dem Rückstand am Hochvakuum. Man erhält 24,6 g 2-Fluor-4-chlor-5-difluormethoxyanilin vom Siedepunkt 72-75°C/0,02 mbar.

<u>Beispiel 2:</u> Herstellung von 2-Fluor-4-chlor-5-(2-chlor-1,1,2-trifluoräthoxy)anilin (Ausgangsmaterial)

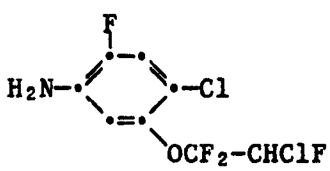

In eine Lösung von 8,1 g (0,05 Mol) 2-Fluor-4-chlor-5-hydroxy-anilin und 1,9 g Kaliumcarbonat in 100 ml Dimethylformamid leitet man unter Rühren bei Raumtemperatur Chlortrifluoräthylen bis zur Sättigung ein. Unter weiterem Einleiten wird das allmählich dunkel werdende Reaktionsgemisch langsam auf eine Temperatur von 80° erwärmt und eine Stunde ausgerührt. Man lässt die dunkelschwarze Lösung auf Raumtemperatur abkühlen und engt sie am Vakuum ein. Der Rückstand wird mit Wasser versetzt und mit Essigsäure-äthylester extrahiert. Die organischen Phasen werden vereinigt, mit gesättigter Kochsalzlösung ausgeschüttelt, über Natriumsulfat getrocknet und am Rotationsverdampfer eingeengt. Man erhält 11,6 g 2-Fluor-4-chlor-5-(2-chlor-1,1,2-trifluoräthoxy) als dunkelbraunes Oel, das direkt weiter umgesetzt wird.

Beispiel 3: Herstellung von N-(2-Fluor-4-chlor-5-difluormethoxy-phenyl)-3,4,5,6-tetrahydrophthalsäureimid

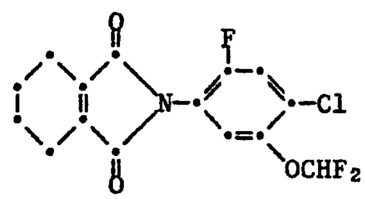

13,1 g (0,062 Mol) 2-Fluor-4-chlor-5-difluor-methoxy-anilin (Beispiel 1) und 9,4 g (0,062 Mol) 3,4,5,6-Tetrahydrophthalsäureanhydrid werden mit 20 ml Propionsäure versetzt und 4 Stunden am Rückfluss gekocht. Nach dem Abkühlen auf Raumtemperatur giesst man die Reaktionslösung auf Eiswasser und extrahiert mit Essigsäureäthylester. Die vereinigtien Extrakte werden mti Wasser neutral gewaschen, über Natriumsulfat getrocknet und eingedampft. Der ölige Rückstand wird aus Diisopropyläther kristallisiert. Man erhält so 11,3 g Titelprodukt mit einem Schmelzpunkt von 105-107°.

Beispiel 4: Herstellung von N-[2-Fluor-4-chlor-5-(2-chlor-1,1,2-trifluoräthoxy)phenyl]-3,4,5,6-tetrahydro-phthalsäureimid

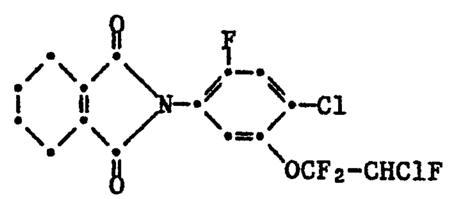

Eine Lösung von 11,6 g (0,042 Mol) des im Beispiel 2 erhaltenen 2-Fluor-4-chlor-5-(2-chlor-1,1,2-trifluoräthoxy)anilins in 120 ml Tetrahydrofuran wird mit 6,5 g 3,4,5,6-Tetrahydrophthalsäureanhydrid

versetzt und 1 Stunde bei Raumtemperatur gerührt. Das Lösungsmittel wird dann am Rotationsverdampfer abdestilliert und der Rückstand in 20 ml Propionsäure aufgenommen und 4 Stunden am Rückfluss gekocht. Nach dem Abkühlen wird das Reaktionsgemisch aus Eiswasser gegossen und mit Essigsäure-äthylester extrahiert. Die organische Phase wird mit Wasser neutral gewaschen, über Natriumsulfat getrocknet, filtriert und eingedampft. Das zürückbleibende Oel wird in Diisopropyläther aufgenommen und zur Kristallisation gebracht. Man erhält so 5.9 g Titelprodukt, welches bei 88-90° schmilzt.

Beispiel 5: Herstellung von N-[2-Fluor-4-chlor-5-(2-chlorprop-2-en-1-yloxy)-phenyl]-3,4,5,6-tetrahydrophthalsäureimid

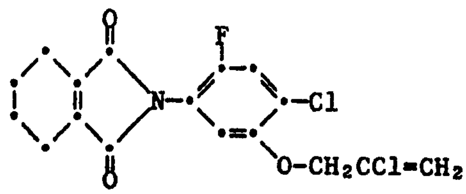

7,1 g (0,024 Mol) N-(2-Fluor-4-chlor-5-hydroxy)-phenyl-3,4,5,6-tetrahydrophthalimid werden in 50 ml Methyläthylketon gelöst. Nach Zugabe von 4,1 g (0,03 Mol) Kaliumcarbonat heizt man zum Rückfluss auf, wobei bereits nach 10 Minuten das Kaliumsalz als dicke, weisse Masse ausfällt. Man gibt 3,5 ml (0,045 Mol) 2,3-Dichlorpropen-1 zu und lässt drei Stunden am Rückfluss rühren. Dann filtriert man ab und dampft das Filtrat ein. Das zurückbleibende, braune Oel chromatographiert man über Kieselgel mit Petroläther/Essigester 3:1. Nach dem Eindampfen der Hauptfraktionen erhält man das Produkt als hellgelbes, klares Oel. Dieses kristallisiert durch und man erhält 4,8 g N-[2-Fluor-4-chlor-5-(2-chlorprop-2-enyl-1-oxy)phenyl]-3,4,5,6-tetrahydrophthalsäureimid mit einem Schmelzpunkt von 88-89°C.

Tabelle 1

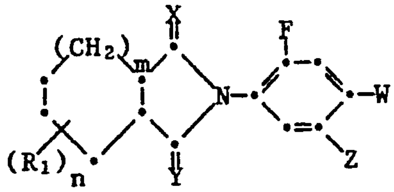

| No. | $(R_1)_n$ | m | X | Y | W | Z | Smp. |
|---|---|---|---|---|---|---|---|
| 1.001 | - | 1 | O | O | Cl | $OCHF_2$ | 105-107° |
| 1.002 | - | 1 | O | O | Cl | $OCF_2CHClF$ | 88-90° |
| 1.003 | - | 1 | O | O | Cl | $OCH_2CCl=CH_2$ | 88-89° |
| 1.004 | - | 1 | O | O | Cl | $OCF_3$ | |
| 1.005 | - | 1 | O | O | Cl | $SCF_3$ | |
| 1.006 | - | 1 | O | O | Cl | $SCHF_2$ | |
| 1.007 | - | 1 | O | O | Cl | $OCF_2CHF_2$ | 93-94° |
| 1.008 | - | 1 | O | O | Cl | $OCCl=CHCl$ | 122-124° |
| 1.009 | - | 1 | O | O | Cl | $OCH_2C\equiv CI$ | |
| 1.010 | - | 1 | O | O | Cl | $OCH_2CCl=CHCl$ | |
| 1.011 | - | 1 | O | O | Cl | $OCH(CH_3)CH_2Cl$ | |
| 1.012 | - | 1 | O | O | Cl | $OCH_2CH_2F$ | |
| 1.013 | - | 1 | O | O | Cl | $OCH_2CH_2Cl$ | |
| 1.014 | - | 1 | O | O | Cl | $OCH_2CH_2Br$ | |
| 1.015 | - | 1 | O | O | Cl | $OCH_2CH_2I$ | |
| 1.016 | - | 1 | O | O | Cl | $OCF_2CHFCF_3$ | Oel |
| 1.017 | - | 1 | O | O | Cl | $OCF_2CHCl_2$ | |
| 1.018 | - | 1 | O | O | Cl | $OCF_2CF_3$ | 75-77° |
| 1.019 | - | 1 | O | O | Cl | $OCH_2CH=CHCl$ | 63-66° (cis) |
| 1.020 | - | 1 | O | O | Cl | $OCH_2CH=CHCl$ | 126-127° (trans) |
| 1.021 | - | 1 | O | O | Cl | $OCH_2CBr=CH_2$ | 95-96° |
| 1.022 | - | 1 | O | O | Cl | $OCH_2\equiv CCH_2Cl$ | 112-114° |
| 1.023 | - | 1 | O | O | Cl | $OCClF_2$ | 98-100° |

<u>**Tabelle 2**</u>

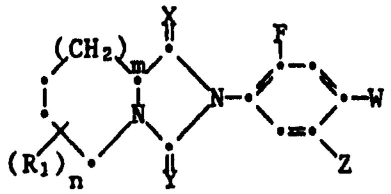

| No. | $(R_1)_n$ | m | X | Y | W | Z | Smp. |
|---|---|---|---|---|---|---|---|
| 2.001 | - | 1 | O | O | Cl | $OCHF_2$ | 83-85° |
| 2.002 | - | 1 | O | O | Cl | $OCF_2CHClF$ | |
| 2.003 | - | 1 | O | O | Cl | $OCH_2CCl{=}CH_2$ | |
| 2.004 | - | 1 | O | O | Cl | $OCF_3$ | |
| 2.005 | - | 1 | O | O | Cl | $SCF_3$ | |
| 2.006 | - | 1 | O | O | Cl | $SCHF_2$ | |
| 2.007 | - | 1 | O | O | Cl | $OCF_2CHF_2$ | |
| 2.008 | - | 1 | O | O | Cl | $OCCl{=}CHCl$ | |
| 2.009 | - | 1 | O | O | Cl | $OCH_2C{\equiv}CI$ | |
| 2.010 | - | 1 | O | O | Cl | $OCH_2CCl{=}CH_2Cl$ | |
| 2.011 | - | 1 | O | O | Cl | $OCH(CH_3)CHCl_2$ | |
| 2.012 | - | 1 | O | O | Cl | $OCH_2CH_2F$ | |
| 2.013 | - | 1 | O | O | Cl | $OCH_2CH_2Cl$ | |
| 2.014 | - | 1 | O | O | Cl | $OCH_2CH_2Br$ | |
| 2.015 | - | 1 | O | O | Cl | $OCH_2CH_2I$ | |
| 2.016 | - | 1 | O | O | Cl | $OCF_2CHFCF_3$ | |
| 2.017 | - | 1 | O | O | Cl | $OCF_2CHCl_2$ | |
| 2.018 | - | 1 | O | O | Cl | $OCF_2CF_3$ | |
| 2.201 | - | 0 | O | O | Cl | $OCHF_2$ | 94-97° |
| 2.202 | - | 0 | O | O | Cl | $OCF_2CHClF$ | |
| 2.203 | - | 0 | O | O | Cl | $OCH_2CCl{=}CH_2$ | 104-106° |
| 2.204 | - | 0 | O | O | Cl | $OCF_3$ | |
| 2.205 | - | 0 | O | O | Cl | $SCF_3$ | |
| 2.206 | - | 0 | O | O | Cl | $SCHF_2$ | |
| 2.207 | - | 0 | O | O | Cl | $OCF_2CHF_2$ | |
| 2.208 | - | 0 | O | O | Cl | $OCCl{=}CHCl$ | |
| 2.209 | - | 0 | O | O | Cl | $OCH_2C{\equiv}CI$ | |
| 2.210 | - | 0 | O | O | Cl | $OCH_2CCl{=}CHCl$ | |
| 2.211 | - | 0 | O | O | Cl | $OCH(CH_3)CH_2Cl$ | |

Tabelle 2 (Fortsetzung)

| No. | $(R_1)_n$ | m | X | Y | W | Z | Smp. |
|---|---|---|---|---|---|---|---|
| 2.212 | - | 0 | 0 | 0 | Cl | $OCH_2CH_2F$ | |
| 2.213 | - | 0 | 0 | 0 | Cl | $OCH_2CH_2Cl$ | |
| 2.214 | - | 0 | 0 | 0 | Cl | $OCH_2CH_2Br$ | |
| 2.215 | - | 0 | 0 | 0 | Cl | $OCH_2CH_2I$ | |
| 2.216 | - | 0 | 0 | 0 | Cl | $OCF_2CHFCF_3$ | |
| 2.217 | - | 0 | 0 | 0 | Cl | $OCF_2CHCl_2$ | |
| 2.218 | - | 0 | 0 | 0 | Cl | $OCF_2CF_3$ | |

Tabelle 3:

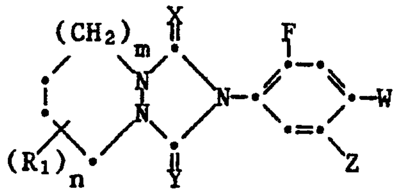

| No. | $(R_1)_n$ | m | X | Y | W | Z | Smp. |
|---|---|---|---|---|---|---|---|
| 3.001 | - | 1 | 0 | 0 | Cl | $OCHF_2$ | 162-164° |
| 3.002 | - | 1 | 0 | 0 | Cl | $OCF_2CHClF$ | |
| 3.003 | - | 1 | 0 | 0 | Cl | $OCH_2CCl{=}CH_2$ | |
| 3.004 | - | 1 | 0 | 0 | Cl | $OCF_3$ | |
| 3.005 | - | 1 | 0 | 0 | Cl | $SCF_3$ | |
| 3.006 | - | 1 | 0 | 0 | Cl | $SCHF_2$ | |
| 3.007 | - | 1 | 0 | 0 | Cl | $OCF_2CHF_2$ | |
| 3.008 | - | 1 | 0 | 0 | Cl | $OCCl{=}CHCl$ | |
| 3.009 | - | 1 | 0 | 0 | Cl | $OCH_2C{\equiv}CI$ | |
| 3.010 | - | 1 | 0 | 0 | Cl | $OCH_2CCl{=}CHCl$ | |
| 3.011 | - | 1 | 0 | 0 | Cl | $OCH(CH_3)CH_2Cl$ | |
| 3.012 | - | 1 | 0 | 0 | Cl | $OCH_2CH_2F$ | |
| 3.013 | - | 1 | 0 | 0 | Cl | $OCH_2CH_2Cl$ | |
| 3.014 | - | 1 | 0 | 0 | Cl | $OCH_2CH_2Br$ | |
| 3.015 | - | 1 | 0 | 0 | Cl | $OCH_2CH_2I$ | |
| 3.016 | - | 1 | 0 | 0 | Cl | $OCF_2CHFCF_3$ | |
| 3.017 | - | 1 | 0 | 0 | Cl | $OCF_2CHCl_2$ | |
| 3.018 | - | 1 | 0 | 0 | Cl | $OCF_2CF_3$ | |

Tabelle 3: (Fortsetzung)

| No. | $(R_1)_n$ | m | X | Y | W | Z | Smp. |
|---|---|---|---|---|---|---|---|
| 3.301 | - | 0 | 0 | 0 | Cl | $OCHF_2$ | 140-141° |
| 3.302 | - | 0 | 0 | 0 | Cl | $OCF_2CHClF$ | |
| 3.303 | - | 0 | 0 | 0 | Cl | $OCH_2CCl=CH_2$ | |
| 3.304 | - | 0 | 0 | 0 | Cl | $OCF_3$ | |
| 3.305 | - | 0 | 0 | 0 | Cl | $SCF_3$ | |
| 3.306 | - | 0 | 0 | 0 | Cl | $SCHF_2$ | |
| 3.307 | - | 0 | 0 | 0 | Cl | $OCF_2CHF_2$ | |
| 3.308 | - | 0 | 0 | 0 | Cl | OOCl=CHCl | |
| 3.309 | - | 0 | 0 | 0 | Cl | $OCH_2C\equiv CI$ | |
| 3.310 | - | 0 | 0 | 0 | Cl | $OCH_2CCl=CHCl$ | |
| 3.311 | - | 0 | 0 | 0 | Cl | $OCH(CH_3)CHCl_2$ | |
| 3.312 | - | 0 | 0 | 0 | Cl | $OCH_2CH_2F$ | |
| 3.313 | - | 0 | 0 | 0 | Cl | $OCH_2CH_2Cl$ | |
| 3.314 | - | 0 | 0 | 0 | Cl | $OCH_2CH_2Br$ | |
| 3.315 | - | 0 | 0 | 0 | Cl | $OCH_2CH_2I$ | |
| 3.316 | - | 0 | 0 | 0 | Cl | $OCF_2CHFCF_3$ | |
| 3.317 | - | 0 | 0 | 0 | Cl | $OCF_2CHCl_2$ | |
| 3.318 | - | 0 | 0 | 0 | Cl | $OCF_2CF_3$ | |

Formulierungsbeispiele:

Beispiel 6: Formulierungsbeispiele für Wirkstoffe der Formel I (% = Gewichtsprozent)

a) Spritzpulver

| | a) | b) | c) |
|---|---|---|---|
| Wirkstoff gemäss Tabelle 1 bis 3 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 bis 3 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 bis 3 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabelle 1 bis 3 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabelle 1 bis 3 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabelle 1 bis 3 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff gemäss Tabelle 1 bis 3 | 5 % |
| Isopropylamin | 1 % |
| Octylphenolpolyäthylenglykoläther (78 Mol AeO) | 3 % |
| Wasser | 91 % |

Biologische Beispiele:

Beispiel 7: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25°C und 50-70 % relativer Luftfeuchtigkeit gehalten und der Versuch nach 3 Wochen ausgewertet.

In diesem Versuch zeigen die geprüften Verbindungen der Tabellen 1 bis 3 starke Herbizidwirkung.

Beispiel 8: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die geprüften Verbindungen der Tabellen 1 bis 3 starke bis sehr starke Herbizidwirkung.

Beispiel 9: Herbizidwirkung vor dem Auflaufen der Pflanzen

Kunststofföpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase

von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet. Die geprüften Verbindungen der Tabellen 1 bis 3 zeigen dabei gute bis sehr gute Herbizidwirkung.

<u>Beispiel 10:</u> <u>Herbizidwirkung für Wasserreis</u> (paddy)
Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 bis 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die geprüftenVerbindungen der Tabellen 1 bis 3 schädigen dabei die Unkräuter, nicht aber den Reis.

<u>Beispiel 11:</u> <u>Wuchshemmung bei tropischen Bodenbedecker-Leguminosen (cover crops).</u>
Die Versuchspflanzen (Centrosema plumieri und Centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem

Versuch zeigen die mit Wirkstoffen der Tabellen 1 bis 3 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 12: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die geprüften Wirkstoffe der Tabellen 1 bis 3 eine merkliche Erhöhung der Anzahl und des Gewichts der Schoten im Haupttrieb.

Beispiel 13: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabellen 1 bis 3 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 14: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach

Bedarf bewässert. Die aufgeleufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1 bis 3 besprüht. Die Wirkstoffmenge beträgt umgerechnet bis zu 500 g Aktivsubstanz pro Hektar. 21 Tage nach Applikation wird das Wachstum der Gräser beurteilt.

Die behandelten Pflanzen zeigten eine Reduzierung des Neuzuwachses von um 10-30 % im Vergleich zur unbehandelten Kontrolle.

Beispiel 15: Desiccations- und Defoliationswirkung

Im Gewächshaus wurden Baumwollpflanzen der Sorte "Deltapine" in Tontöpfen angezogen. Nach abgeschlossener Kapselbildung wurden sie mit wässerigen Zubereitungen des zu prüfenden Wirkstoffes in einer Aufwandmenge, die 1,2, 0,6 und 0,3 kg/ha im Feld entspräche, gespritzt. Unbehandelte Pflanzen wurdne als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch liessen Verbindungen der Tabellen 1-3 bei Aufwandmengen von 0,6 und 1,2 kg/ha nach 7 Tagen bloss noch wenig vertrocknete Blätter auf den Pflanzen (mehr als 80 % Blattfall und Austrocknung.

Patentansprüche:

1. N-(2-Fluorphenyl)-azolidine der Formel I

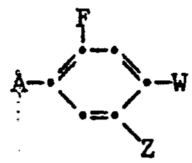

(I)

worin

A einen der bicyclischen Reste

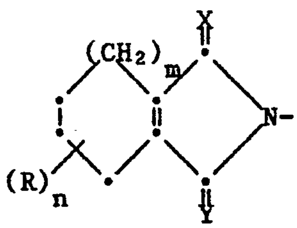 , 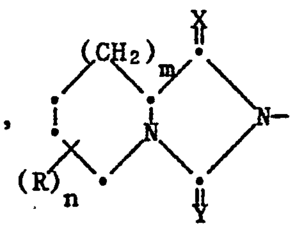 oder 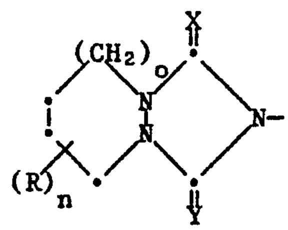

m Null oder eins

n Null eins oder zwei

o Null, eins, zwei oder drei,

R $C_1$-$C_4$ Alkyl oder Halogen

W Halogen

X und Y unabhängig voneinander Sauerstoff oder Schwefel

Z $C_1$-$C_4$ Halogenalkoxy, $C_1$-$C_4$ Halogenalkylthio, $C_2$-$C_6$ Halogenalkenyloxy, $C_2$-$C_6$ Halogenalkenylthio, $C_3$-$C_6$ Halogenalkinyloxy oder $C_3$-$C_6$ Halogenalkinylthio

bedeuten.

2. N-(2-Fluorphenyl)-azolidine gemäss Anspruch 1 der Formel Ia

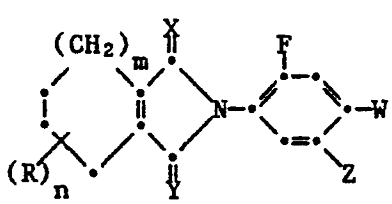

Ia

worin m Null oder eins bedeutet und n, R, W, X, Y und Z die im Anspruch 1 gegebene Bedeutung haben.

3. N-(2-Fluorophenyl)-azolidine gemäss Anspurch 2 der Formel Ia, worin m eins, n Null, W Chlor oder Brom, X und Y je Sauerstoff und Z $C_1$-$C_4$ Halogenalkyl, $C_1$-$C_4$ Halogenalkylthio, $C_3$-$C_6$ Halogenalkenyloxy, $C_3$-$C_6$ Halogenalkenylthio, $C_3$-$C_6$ Halogenalkinyloxy oder $C_3$-$C_6$ Halogenalkinylthio bedeutet.

4. N-(2-Fluor-4-chlor-5-difluormethoxy-phenyl)-3,4,5,6-tetrahydrophthalsäureimid gemäss Anspruch 1.

5. N-[2-Fluor-4-chlor-5-(2-chlor-1,1,2-trifluoräthoxy)-phenyl]-3,4,5,6-tetrahydro-phthalsäureimid gemäss Anspruch 1.

6. N-[2-Fluor-4-chlor-5-(2-chlorprop-2-en-1-yloxy)phenyl]-3,4,5,6-tetrahydrophthalsäureimid gemäss Anspruch 1.

7. N-(2-Fluorphenyl)-azolidine gemäss Anspruch 1 der Formel Ib

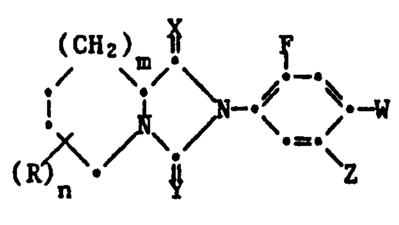

(Ib)

worin m Null oder eins bedeutet und n, R, W, X, Y und Z die im Anspruch 1 gegebene Bedeutung haben.

8. 2-Fluorophenyl-azolidine gemäss Anspruch 7 der Formel Ib, worin m eins, n Null, W Chlor oder Brom, X und Y je Sauerstoff und Z $C_1$-$C_6$ Halogenalkoxy, $C_1$-$C_6$ Halogenalkylthio, $C_3$-$C_6$ Halogenalkenyloxy, $C_3$-$C_6$ Halogenalkenylthio, $C_3$-$C_6$ Halogenalkinyloxy oder $C_3$-$C_6$ Halogenalkinylthio bedeuten.

9. N-(2-Fluorophenyl)-azolidine gemäss Anspruch 1 der Formel Ic

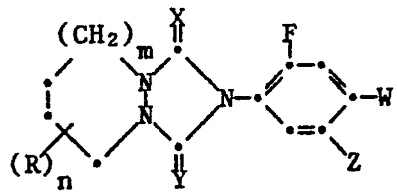

(Ic)

worin m Null oder 1 bedeutet und n, R, W, X, Y und Z die im Anspruch 1 gegebene Bedeutung haben.

10. N-(2-Fluorophenyl)-azolidine gemäss Anspruch 9 der Formel Ic, worin m eins, n Null, W Chlor, X und Y je Sauerstoff und Z $C_1$-$C_6$ Halogenalkoxy, $C_1$-$C_6$ Halogenalkylthio, $C_3$-$C_6$ Halogenalkenyloxy, $C_3$-$C_6$ Halogenalkenylthio, $C_3$-$C_6$ Halogenalkinyloxy oder $C_3$-$C_6$ Halogenalkinylthio bedeuten.

11. Verfahren zur Herstellung der N-(2-Fluorphenyl)-azolidinen der Formel Ia gemäss Anspruch 2, dadurch gekennzeichnet, dass man das Anhydrid einer bicyclischen Dicarbonsäure entsprechend der Formel II

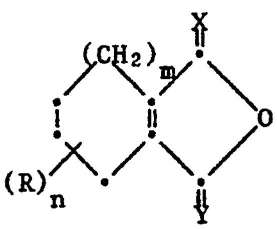

,

(II)

worin m, n, o, R, X und Y die im Anspruch 1 gegebene Bedeutung haben, gegebenenfalls in einem inerten organischen Lösung- oder Verdünnungsmittel mit der äquimolaren Menge eines 2-Fluoranilins der Formel III kondensiert

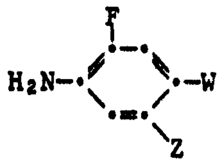

(III),

worin W und Z die in Anspruch 1 gegebene Bedeutung haben, und das entstandene Amid der Formel IV

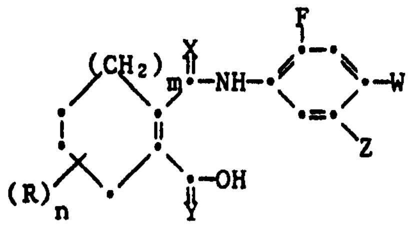

(IV)

worin m, n, o, R, W, X, Y und Z die unter der Formel I gegebene Bedeutung haben, gegebenenfalls ohne es aus dem Reaktionsgemisch zu isolieren bei Temperaturen zwischen 80 und 230°C ringschliesst.

12. Verfahren zur Herstellung der N-(2-Fluorphenyl)-azolidine der Formel Ia gemäss Anspruch 2, dadurch gekennzeichnet, dass man ein Säureanhydrid der Formel IIa

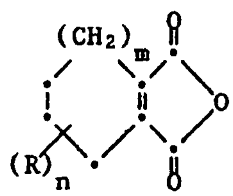

(IIa)

worin m Null oder eins bedeutet und n, R und W die unter der Formel I gegebene Bedeutung haben, gegebennfalls in einem inerten organischen Lösungsmittel mit der äquimolaren Menge eines 2-Fluoranilins der Formel III kondensiert

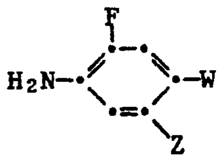

(III)

worin W und Z die unter der Formel I gegebene Bedeutung haben, und das entstandene Amid der Formel IVa

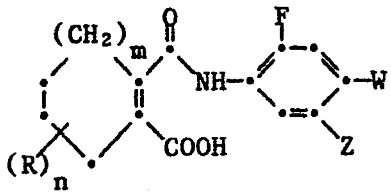

(IVa)

worin m Null oder eins bedeutet und n, R, W und Z die unter der Formel I gegebene Bedeutung haben, gegebenenfalls ohne es aus dem Reaktionsgemisch zu isolieren, bei einer Temperatur von 80-230°C ringschliesst.

13. Eine Verbindung der Formel IVa

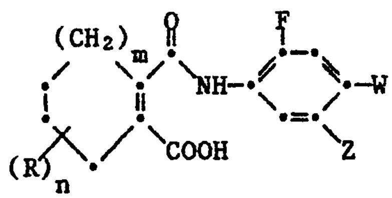

(IVa)

worin m eins und n Null bedeuten während W und Z die im Anspruch 1 gegebene Bedeutung haben, als Zwischenprodukt zur Herstellung der N-(2-Fluorphenyl)azolidine gemäss Anspruch 1.

14. Ein herbizides und Pflanzenwachstum regulierendes Mittel, dadurch gekennzeichnet, dass es neben Träger- und/oder anderen Zuschlagstoffen als Wirkstoff ein N-(2-Fluorphenyl)-azolidin der Formel I, Anspruch 1 enthält.

15. Die Verwendung eines Wirkstoffes gemäss Anspruch 1, oder einen solchen Wirkstoff enthaltendes Mittel zur Bekämpfung unerwünschten Pflanzenwachstums.

16. Die Verwendung eines Wirkstoffes gemäss Anspruch 1 oder einen solchen Wirkstoff enthaltendes Mittel zur Hemmung des Pflanzenwachstums.

17. Die Verwendung eines Wirkstoffes gemäss Anspruch 1, oder einen solchen Wirkstoff enthaltendes Mittel zur Beeinflussung des Pflanzenwachstums zum Zweck der Ertragssteigerung.

18. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Wirkstoffes der Formel I, Anspruch 1 oder eines eine solche Verbindung enthaltenden Mittels behandelt.

19. Verfahren zur Unterdrückung des Pflanzenwachstums über das 2-Blattstadium hinaus, dadurch gekennzeichnet, dass man die Pflanzen während ihres Wachstums mit einer wirksamen Menge eines Wirkstoffes der Formel I Anspruch 1 oder eines einen solchen Wirkstoff enthaltenden Mittels behandelt.

FO 7.5/NU/cc*/sm*